# EUROPEAN PATENT APPLICATION

(11) **EP 4 570 170 A2**
(43) Date of publication of application: **18.06.2025**
(21) Application number: 25174636.8
(22) Date of filing: 23.10.2023
(51) Int. Cl.: A61B 5/00

(54) **METHOD OF MEASURING A FLUORESCENCE SIGNAL AND OF DETERMINING A 3D REPRESENTATION, IMAGE CAPTURING AND PROCESSING DEVICE**

(30) Priority: 15.11.2022 US 202263425397 P
(62) Divisional of application: 23205174.8
(71) Applicant: Quest Photonic Devices B.V., 1771 SR Wieringerwerf (NL)
(72) Inventor: HOVELING, Richelle Johanna Maria, 1623 JA Hoorn (NL); KOOPMAN, Thomas, 1013 WR Amsterdam (NL); MEESTER, Richard Johannes Cornelis, 1771 DA Wieringerwerf (NL)
(74) Representative: Seemann & Partner Patentanwälte mbB

(57) **Abstract**

An image capturing and processing device (2) configured to measure a fluorescence signal in a tissue of a limb (4), to which a fluorescent agent (8) has been added, and of determining a 3D representation of at least a section of the limb (4). The device (2) comprising an illumination unit (16), a fluorescence imaging unit (22) and a topology capturing unit. The topology capturing unit is configured to capture data on a topology of the surface of at least the section of the limb (4) and to determine a 3D representation of at least the section of the limb (4) from the captured data. An output unit is configured to output the fluorescence image (7) and a visualization of the 3D representation.

## Description

The invention relates to an image capturing and processing device configured to measure a fluorescence signal in a tissue of a limb to which a fluorescent agent has been added and to determine a 3D representation of at least a section of the limb, wherein the tissue to which the fluorescent agent has been added forms part of the limb.

The image capturing and processing device relates to imaging and measuring of the lymphatic function, in particular in view of the diagnosis, treatment and/or prevention of lymphedema.

Lymphedema is an accumulation of lymphatic fluid in the body's tissue. While oxygenated blood is pumped via the arteries from the heart to the tissue, deoxygenated blood returns to the heart via the veins. Because the pressure level on the arterial side is much higher than on the vein side, a colorless fluid part of the blood is pushed into the space between the cells. Typically, more fluid is pushed out, than reabsorbed on the vein side. The excess fluid is transported by the lymphatic capillaries. Furthermore, the fluid carries away local and foreign substances such as larger proteins and cellular debris. Once in the lymphatic system, this fluid including the transported substances is referred to as lymph or lymph fluid.

The lymphatic system comprises lymphatic vessels having one way valves similar to vein valves for transporting the lymph to the next lymph node. The lymph node performs removal of certain substances and cleans the fluid before it drains back to the blood stream.

If the lymphatic system becomes obstructed in that the lymph flow is blocked or not performed at the desired level, the lymph fluid accumulates in the interstitial space between the tissue cells. This accumulation, which is due to an impairment of lymphatic transport, is called lymphedema. The accumulation of lymph can cause inflammatory reaction which damages the cells surrounding the affected areas. It can further cause fibrosis which can turn into a hardening of the affected tissue.

Because lymphedema is a lifelong condition for that no cure or medication exists, early diagnoses and appropriate early counter measures for improving drainage and reducing the fluid load are of high importance for patient's well-being and recovering. Possible treatments such as lymphatic massage and compression bandages up to surgery depend on the level of severity, which is a four stage system defined by the World Health Organization (WHO) as follows:

| | |
|---|---|
| Stage 1: | a normal flow in the lymphatic system. No signs or symptoms. |
| Stage 2: | accumulation of fluid with swelling. |
| Stage 3: | permanent swelling that does not resolve with elevation of the affected limb or body part. |
| Stage 4: | elephantiasis (large deformed limb), skin thickening with "wart like" growth and extensive scarring. |

For diagnosis of the function of the lymphatic system, commonly used techniques are a manual inspection of the affected limb or body part by a physician. A known imaging technique is lymphoscintigraphy. In this technique, a radio tracer is injected in the tissue of the affected body part and subsequently MRI (Magnetic Resonance Imaging), CT (Computer Tomography), a PET-CT-scan (Positron Emission Tomography) or ultrasound imaging is performed.

A relatively new imaging technique is infrared fluorescence imaging using a fluorescent dye, for example ICG (Indocyanine Green). ICG is a green colored medical dye that is used for over 40 years. The dye emits fluorescent light when exited with near infrared light having a wavelength between 600 nm and 800 nm. Due to this excitation, ICG emits fluorescence light between 750 nm and 950 nm. The fluorescence of the ICG dye can be detected using a CCD or CMOS sensor or camera. The fluorescent dye is administered to the tissue of an affected limb or body part and the concentration and flow of the lymphatic fluid can be traced on the basis of the detected fluorescence light.

US 2020/0214571 A1 discloses a device for taking fluorescence images with different fluorescent dyes. Furthermore, it seems to be disclosed that these images are combined with PET-CT images. US 2020/0061671 A1 discloses an imaging device, with which a visible image, a fluorescence image and a thermal image can be combined. A so-called three dimensional mapping is mentioned without a precise statement made in this regard. US 2015/0198797 A1 discloses a device, with which a perfusion measurement can be performed. In 10 addition to a fluorescence measurement, for example a measurement of ICG, a 3D camera can be used to carry out the perfusion measurements

It is an object of the invention to provide an enhanced image capturing and processing device, wherein in particular an enhanced fluorescence imaging output can be provided.

The object is solved by the subject matter of claim 1. The dependent claims represent advantageous embodiments.

According to an example, there is a method of measuring a fluorescence signal in a tissue of a limb, to which a fluorescent agent has been added, and of determining a 3D representation of at least a section of the limb, wherein the tissue to which the fluorescent agent has been added forms part of the limb, the method comprising the steps of:
capturing a fluorescence image by illuminating the tissue with excitation light having a wavelength suitable to generate emitted light by excited emission of the fluorescent agent, and by spatially resolved measurement of the emitted light so as to provide the fluorescence image,
capturing data on a topology of the surface of at least the section of the limb and determining a 3D representation of at least the section of the limb from the captured data,
outputting the fluorescence image and a visualization of the 3D representation.

Advantageously, the information, which is derivable from the fluorescence image can be analyzed in view of or in combination with a 3D topology of the limb. This enables the physician to base the diagnosis on more information, which can enhance the preciseness and reliability of the diagnosis.

Data on the topology of the surface of the limb can be acquired using a suitable scanner or acquisition device. This can be for example a device for capturing color images, stereo images, 3D images, a line scanner, a LIDAR scanner, a time of flight sensor, a point cloud generator or any other suitable device that can generate a 3D data set from an object. The 3D data set, i.e. the data on the topology of the surface of the patient's limb, is accordingly for example a point cloud or any other 3D representation. For visualization of the 3D representation, the 3D data set can be transformed (i.e. computed) into a point cloud, a grid network structure, a 3D model rendering or any other suitable 3D illustration. It is also possible that the visualization is a true 3D image that is reproduced via a suitable device. The device can be for example a 3D screen. A 3D screen is typically used in combination with suitable glasses, that are worn by a user when watching objects on the 3D screen. The device for reproduction of the 3D image can also be a 3D headset, 3D googles or glasses, VR glasses, AR glasses or the like. The user can thus view and analyze the 3D representation in true 3D.

According to another example, the method further comprises the steps of determining a volume of at least the section of the limb from the 3D representation and outputting the fluorescence image and the visualization of the 3D representation together with a visualization of the determined volume.

A measurement of the volume of the limb is typically used to determine the extent or severity of lymphedema. It can be also used to analyze the success of the therapeutic treatment. If the treatment of lymphedema is effective, the volume of the affected limb should change in any way. In most cases, it is expected that an effective therapy reduces the volume of the affected limb. The traditional way to determine the volume of the limb is the so called tape measurement. This is performed by starting with a point on the foot or hand of the patient and by replacing tapes in regular intervals up the arm or leg. At the respective positions of the tapes, the circumference of the limb is measured. A total volume of the limb is approximated by cylinders or frustums having a circumference that equals the measured circumferences at the respective positions of the tapes. This traditional method however suffers from significant disadvantages. Firstly, the determination of the volume is a very rough approximation. Secondly, it is very difficult to create reproducible measurement results, in particular if the different measurements are performed by different operators or at different points in time.

The method exactly removes these disadvantages by providing a precise and reproducible determination of the volume of the limb, because the determination of the volume is based on measured 3D data. It is therefore possible to observe changes of the volume of the limb over time. An onset of lymphedema can be detected and the success of the therapeutic treatment or an individual response of the patient to a particular treatment can be determined.

Based on the determination of the volume of the limb, which is performed with the method, it is also possible to compare a left and right limb of the patient. Comparing the volume of the corresponding limbs is a well-known indicator for diagnosing whether or not one of the two limbs is affected by lymphedema or not. For example, the left arm and the right arm or the left leg and the right leg of a patient can compared. Furthermore, absolute values of the volumes can be compared. This can be advantageous because a difference between the volumes of the two limbs can be for example analyzed in view of the total volume of one limb. The measurement result can be something like: the volume of the left arm is 10% higher than the volume of the right arm.

In combination with the 3D representation of the limb, a volume distribution can be generated. For example, the value for the volume over the longitudinal extension of the limb can be calculated. This allows the physician to detect certain parts of the limb that seem to be affected by lymphedema. This measurement can be combined with the fluorescence image. The physician can verify or falsify the preliminary diagnosis, wherein the diagnosis can advantageously be based on completely new criteria. This enhances the quality of the diagnosis, which does no longer comprise a personal component that is due to the individual experience and qualification of the physician, in contrast to this, the result is based on an objective measurement.

According to another example, the method further comprising the steps of: superimposing the fluorescence image and the visualization of the 3D representation of at least the section of the limb so as to provide an overlay image and outputting the overlay image as the output of the fluorescence image and the visualization of the 3D representation.

The output of the fluorescence image and the visualization of the 3D representation can be performed by displaying for example the two images side-by-side on a screen or monitor. Objects which will be visible in the fluorescence image can be found at the same position in the visualization of the 3D representation. This can be done by simply comparing the structures that can be seen at corresponding positions in the two images. This process can however be further simplified by using an overlay image. The overlay image significantly simplifies the allocation of corresponding parts in the 3D representation and in the fluorescence image. The overlay image therefore provides the user with a quick and easy way to understand information.

According to still another example, the method is further enhanced in that the steps of capturing the fluorescence image and of capturing data on the topology of the surface of at least the section of the limb are performed simultaneously.

In particular, a fluorescence imaging unit, which captures the fluorescence image, and a topology capturing unit, which captures the data on the topology of the surface, are arranged in one single device. Furthermore, the units are configured in that the acquisition of the image data and the acquisition of topology data can be performed simultaneously.

According to still another example, the method is further enhanced in that the steps of capturing the fluorescence image and data on the topology of the surface of at least the section of the limb and the determination of the 3D representation of at least the section of the limb from the captured data, are performed in at least a first measurement series and a second measurement series, wherein the different measurement series are performed on different limbs or at different points in time, and wherein the step of outputting includes outputting the fluorescence images and the visualizations of the 3D representations of the first and second series, wherein in particular the fluorescence images and the visualizations of the 3D representation of the first and second series are output as at least one difference image.

For example, a first series can be captured at a first examination appointment of a patient. The second series is captured while the same patient returns to a second examination appointment. By comparing the two series of data, the development or process of lymphedema can be observed or the success of a treatment can be evaluated.

In an alternative application scenario, the first series of data can be captured for example on the left arm of a patient. The second series is captured on the right arm of the patient. Typically, lymphedema affects only one of two limbs, for example the left arm but not the right arm. By comparing the left and right limb, a level of lymphedema can be assessed.

According to still another example, the method further comprises the steps of: capturing a visible light image of at least the section of the surface of the limb, wherein a viewing direction and/or a perspective of the fluorescence image and the visible light image are linked via a known relationship and the step of outputting includes: outputting the visible light image together with the fluorescence image and the visualization of the 3D representation.

In other words, the data set comprising information on fluorescence and topology is supplemented by a visible light image. The combination with the visible light image can help the user to spot locations at the patient's limb at which a certain phenomenon is detected. This can be easily performed by comparing the visible light image with the real world. The identification of specific spots can be supported by for example marking certain areas on the limb of the patient. These markings will be visible in the visible light image and can assist the physician to pinpoint certain areas of interest.

Within the context of this specification, a "visible light image" is an image of the real world situation. It reproduces an image impression similar to what can be seen by the human eye. Unlike the human eye, the visible light image can be a color image, a greyscale image or even a false color scale plot. The visible light image shows the surface of the limb comprising the tissue to which the fluorescent agent has been administered. If the tissue is arranged at the surface of the limb, the imaging of the surface of the limb includes imaging of the surface of the tissue.

Naturally, capturing of topology data and of the fluorescence image is often limited to a certain section of the limb. In other words, it is impossible to image the full limb, for example the entire arm or leg in one single image. The following example eliminates this drawback. Advantageously, the method further comprises the steps of:
repeating the steps of capturing of the fluorescence image and capturing of the visible light image to provide a series of fluorescence images and a series of visible light images, wherein the capturing of data on the topology of the surface of the limb is performed in at least the section of the limb that is imaged when capturing the series of fluorescence images and the visible light images,
applying a stitching algorithm on the series of visible light images to generate a large visible light image, wherein the stitching algorithm determines and applies a set of stitching parameters,
applying the stitching algorithm on the series of fluorescence images to generate a large fluorescence image, wherein the stitching algorithm applies the set of stitching parameters determined when performing the stitching of the visible light images wherein
the step of outputting includes
outputting the large visible light image together with the large fluorescence image and the visualization of the 3D representation.

Advantageously, two large images showing for example the entire limb can be output and give the user an excellent overview. The stitching process starts on the basis of the visible light images which typically comprise more distinguishing features. These features allow the stitching algorithm to rearrange the images that are planned to be stitched together. For this purpose, the special features have to be found in the two images that have to be stitched together. It is furthermore possible to create such distinguishing features. This can be for example done by applying marks on the surface of the patient's limb. For the fluorescence images, no similar approach is available. In addition to this, the fluorescence images typically lack a great number of significant features. This is why stitching algorithms often have problems when performing stitching on fluorescence images. This disadvantage is completely removed by applying the stitching algorithm with identical parameters on the visible and fluorescence images, wherein the stitching parameters are derived from the visible light image stitching.

According to yet another example, the viewing direction and the perspective of the fluorescence image and the visible light image are identical. In particular, the fluorescence image and the visible light image are captured through one and the same objective lens. It is advantageous for the execution of the stitching algorithm on both data sets, i.e. on the visible light images and on the fluorescence images, when the fluorescence images and the visible light images are captured using a fixed spatial relationship, in particular with respect to a viewing direction and perspective. By capturing the images through one and the same objective lens this prerequisite can easily be complied with.

According to still another example, capturing of the fluorescence image and capturing of the visible light image are performed simultaneously in absence of time switching between a signal of the fluorescence image and the signal of the visible light image. In the absence of time switching, a high frame repeat rate can be achieved. This can be high as for example 60 frames per second or even higher. A high frame rate is often desired for live images. This opens up a field for various applications.

According to still another example, the steps of capturing the fluorescence image, illuminating the tissue with excitation light and simultaneously capturing the visible light image are performed by a single image capturing device. This single image capturing device provides a compact tool that is easy to use.

The method is further enhanced in that the measurement of the fluorescence signal is performed on a tissue, to which at least a first and a second fluorescent agent has been added, wherein the step of capturing the fluorescence image comprises:
capturing a first fluorescence image in a first wavelength range, which is generated by illuminating the tissue with first excitation light having a first wavelength suitable to generate emitted light by a first excited emission of the first fluorescent agent, and
capturing a second fluorescence image in a second wavelength range, which is generated by illuminating the tissue with second excitation light having a second wavelength suitable to generate emitted light by a second excited emission of the second fluorescent agent and the step of outputting comprises:
   outputting the first and the second fluorescence image and the visualization of the 3D representation.

Fluorescence imaging using two fluorescence wavelengths opens up a new field and is a frequent customer need. According to another example, the first fluorescent dye can be for example a methylene blue and the second dye can be ICG. The step of capturing the fluorescence image, according to this example, comprises capturing a first fluorescence image of the fluorescent light emitted by the first fluorescent dye and capturing a second fluorescence image of the fluorescent light emitted by the second fluorescent dye. Capturing of the two images is in particular performed without time switching. The first fluorescence image can be captured in a wavelength range, which is between 700 nm and 800 nm, if methylene blue is used as the first fluorescent dye. The second fluorescence image can be captured in a wavelength range, which is between 800 nm and 900 nm, if ICG is used as the second fluorescent dye. Fluorescence imaging which is based on two different fluorescent agents offers new possibilities for measurements and diagnosis.

The fluorescent dye (the same applies to the first fluorescent dye and to the second fluorescent dye), is for example ICG (Indocyanine Green) or methylene blue. Within the context of this specification, the term "fluorescent dye" or "dye" (also referred to as "fluorochrome" or "fluorophore") refers to a component of a molecule, which causes the molecule to be fluorescent. The component is a functional group in the molecule that absorbs energy of a specific wavelength and re-emits energy at a different specific wavelength. In various aspects, the fluorescent agent comprises a fluorescence dye, an analogue thereof, a derivative thereof, or a combination of these. Appropriate fluorescent dyes include, but are not limited to, indocyanine green (ICG), fluorescein, methylene blue, isosulfan blue, Patent Blue, cyanine5 (Cy5), cyanine5.5 (Cy5.5), cyanine7 (Cy7), cyanine7.5 (Cy7.5), cypate, silicon rhodamine, 5-ALA, IRDye 700, IRDye 800CW, IRDye 800RS, IRDye 800BK, porphyrin derivatives, Illuminare-1, ALM-488, GCP-002, GCP-003, LUM-015, EMI-137, SGM-101, ASP-1929, AVB-620, OTL-38, VGT-309, BLZ-100, ONM-100, BEVA800.

The object is solved by an image capturing and processing device configured to measure a fluorescence signal in a tissue of a limb, to which a fluorescent agent has been added, and to determine a 3D representation of at least a section of the limb, wherein the tissue to which the fluorescent agent has been added forms part of the limb, the device comprising an image capturing device, which comprises:
an illumination unit configured to illuminate the tissue with excitation light having a wavelength suitable to generate emitted light by excited emission of the fluorescent agent,
a fluorescence imaging unit configured to capture a fluorescence image by spatially resolved measurement of the emitted light so as to provide the fluorescence image,
a topology capturing unit configured to capture data on a topology of the surface of at least the section of the limb and to determine a 3D representation of at least the section of the limb from the captured data, wherein the device is enhanced by
a volume determination unit configured to determine a volume of at least the section of the limb from the 3D representation,
the image capturing and processing device further comprising a processing device, which comprises
an output unit configured to output the fluorescence image and a visualization of the 3D representation together with a visualization of the determined volume.

Same or similar advantages which have been mentioned with respect to the method of measuring the fluorescence signal and of determining a 3D representation also apply to the image capturing and processing device in a same or similar way and shall be therefore not be repeated.

The processing device of the image capturing and processing device comprises:
a volume determination unit configured to determine a volume of at least the section of the limb from the 3D representation, and wherein the output unit is further configured to:
output the fluorescence image and the visualization of the 3D representation together with a visualization of the determined volume.

The possibility to determine a 3D representation, a fluorescence signal and a value for the volume of the limb gives the user of the image capturing and processing device an excellent basis for diagnosis of for example lymphedema.

The processing device can be further enhanced in that it additionally comprises: a superimposing unit configured to superimpose the fluorescence image and the visualization of the 3D representation of at least the section of the limb so as to provide an overlay image and the output unit is configured to output the overlay image as the output of the fluorescence image and the visualization of the 3D representation.

Furthermore, the device can be further enhanced in that the fluorescence imaging unit and the topology capturing unit are configured to simultaneously perform capturing of the fluorescence image and capturing of data on the topology of the surface of at least the section of the limb.

The 3D data and the fluorescence data can advantageously be supplemented with visible light image data. Hence, the image capturing device according to aspects of the invention can advantageously further comprise:
a visible light imaging unit configured to capture a visible light image of at least the section of the surface of the limb, wherein the fluorescence imaging unit and the visible light imaging unit are configured in that a viewing direction and/or a perspective of the fluorescence image and the visible light image are linked via a known relationship, and
the output unit is configured to output the visible light image together with the fluorescence image and the visualization of the 3D representation.

Furthermore, the device can be further enhanced in that
the fluorescence imaging unit and the visible light imaging unit are further configured to repeat capturing of the fluorescence image and the visible light image to provide a series of fluorescence images and a series of visible light images, wherein the topology capturing unit is configured to capture data on the topology of the surface of the limb in at least the section of the limb that is imaged when capturing the series of fluorescence images and the visible light images, the processing device further comprising:
a stitching unit configured to apply a stitching algorithm on the series of visible light images to generate a large visible light image of the limb, the stitching algorithm determining and applying a set of stitching parameters, wherein the stitching unit is further configured to apply the stitching algorithm on the series of fluorescence images to generate a large fluorescence image, wherein the stitching algorithm applies the set of stitching parameters determined when performing the stitching of the visible light images, and wherein
the output unit is configured to output the large visible light image together with the large fluorescence image and the visualization of the 3D representation.

According to yet another advantageous embodiment, the superimposing unit can be configured to superimpose the large visible light image and the large fluorescence image to provide an overlay image of the limb. The output unit can be further configured to output the overlay image together with the visualization of the 3D representation.

According to still another advantageous embodiment, the device can be further enhanced in that the fluorescence imaging unit and the visible light imaging unit are configured in that the viewing direction and the perspective of the fluorescence image and the visible light image are identical, wherein in particular the fluorescence imaging unit and the visible light imaging unit are configured in that the fluorescence image and the visible light image are captured through one and the same objective lens.

Furthermore, the device is advantageously enhanced in that the fluorescence imaging unit and the visible light imaging unit are configured to capture the fluorescence image and the visible light image simultaneously, in absence of time-switching between a signal of the fluorescence image and a signal of the visible light image.

According to still another advantageous embodiment, the image capturing device is further enhanced by comprising: a dichroic prism assembly configured to receive fluorescent light forming the fluorescence image and visible light forming the visible light image through an entrance face, comprising: a first prism, a second prism, a first compensator prism located between the first prism and the second prism,
a further dichroic prism assembly for splitting the visible light in three light components, and a second compensator prism located between the second prism and the further dichroic prism assembly,
wherein the first prism and the second prism each have a cross section with at least five corners, each corner having an inside angle of at least 90 degrees, wherein the corners of the first prism and the second prism each have a respective entrance face and a respective exit face, and are each designed so that an incoming beam which enters the entrance face of the respective prism in a direction parallel to a normal of said entrance face is reflected twice inside the respective prism and exits the respective prism through its exit face parallel to a normal of said exit face,
wherein the normal of the entrance face and the normal of the exit face of the respective prism are perpendicular to each other; and
wherein, when light enters the first prism through the entrance face, the light is partially reflected towards the exit face of the first prism thereby travelling a first path length from the entrance face of the first prism to the exit face of the first prism, and the light partially enters the second prism via the first compensator prism and is partially reflected towards the exit face of the second prism, thereby travelling a second path length from the entrance face of the first prism to the exit face of the second prism,
wherein the first prism is larger than the second prism so that the first and the second path lengths are the same.

Advantageously, the above-referred five prism assembly allows to capture two fluorescence imaging wavelengths and the three colors for visible light imaging, for example red, blue and green. The five prism assembly is advantageous in that the optical path of the light traveling from the entrance surface to a respective one of the sensors have identical length. Hence, all sensors are in focus and furthermore, there is no timing gap between the signals of the sensors. Advantageously, the device does not require time-switching of the received signals. This allows an image capture using a high frame rate and enhanced image quality.

According to still another aspect, the image capturing device defines a first, a second, and a third optical path for directing fluorescence light and visible light to a first, a second, and a third sensor, respectively, the image capturing device further comprises a dichroic prism assembly, configured to receive the fluorescent light and the visible light through an entrance face, the dichroic prism assembly comprising: a first prism, a second prism and a third prism, each prism having a respective first, second, and third exit face, wherein: the first exit face is provided with the first sensor, the second exit face is provided with the second sensor, and the third exit face is provided with the third sensor, wherein in particular the first optical path is provided with a first filter, the second optical path is provided with a second filter, and the third optical path is provided with a third filter, wherein
the first, second, and third filters, in any order, are a green filter, an infrared filter, and a red/blue patterned filter comprising red and blue filters in alternating pattern so that half of the light received by the red/blue patterned filter goes through a blue filter and half of the light received by the red/blue patterned filter goes through a red filter.

Furthermore, according to another aspect, the first, second, and third filters, in any order, are a red/green/blue patterned filter (RGB filter), a first infrared filter, and a second infrared filter, wherein in particular, the first and second infrared filter have different transmission wavelength.

In other words, the first and second infrared filter are for filtering IR-light in different IR wavelength intervals, for example in a first IR-band in which typical fluorescent dyes emit a first fluorescence peak and in a second IR-band in which a typical fluorescent dye emits a second fluorescence peak. Typically, the second IR-band is located at higher wavelength compared to the first IR-band. The first and second infrared filter can also be adjusted to emission bands of different fluorescent agents. Hence, the emission of for example a first fluorescent agent passes the first filter (and is in particular blocked by the second filter) and can be detected on the corresponding first sensor and the emission of the second fluorescent agent passes the second filter (and is in particular blocked by the first filter) and can be detected on the corresponding second sensor. For example, the first filter can be configured to measure the fluorescence emission of methylene blue and the second filter can be configured to measure the fluorescence emission of ICG.

Also with respect to the embodiments, same or similar advantages and advantageous aspects apply, which have been mentioned with respect to the method of measuring the fluorescence signal.

Furthermore, according to an example, there is a method of diagnosing lymphedema, comprising the steps of:
administering a fluorescent agent to a limb,
measuring a fluorescence signal in a tissue of the limb, to which the fluorescent agent has been administered, and determining a 3D representation of at least a section of the limb, wherein the tissue to which the fluorescent agent has been added forms part of the limb,
capturing a fluorescence image by illuminating the tissue with excitation light having a wavelength suitable to generate emitted light by excited emission of the fluorescent agent, and by spatially resolved measurement of the emitted light so as to provide the fluorescence image,
capturing data on a topology of the surface of at least the section of the limb and determining a 3D representation of at least the section of the limb from the captured data,
outputting the fluorescence image and a visualization of the 3D representation,
deriving a diagnostic result relative to lymphedema, in particular relative to a severity or level of lymphedema, by analyzing the fluorescence image and the visualization of the 3D representation.

The method of diagnosing lymphedema can be advantageously performed with higher precision and reliability and therefore provides better results. This entirely new approach can advantageously replace the classical way of diagnosing lymphedema. The traditional way to diagnose lymphedema is to perform a manual inspection of the affected limbs by a physician. This method of performing the diagnosis, however, inevitably includes a non-reproducible and random component, which is due to the individual experience and qualification of the physician. Furthermore, the method of diagnosing lymphedema includes same or similar advantages, which have been previously mentioned with respect to the method of measuring the fluorescent signal. Furthermore, the method provides better results than traditional methods. In particular, the imprecise tape method can be dispensed with.

According to an example, the visualization of the 3D representation can be supplemented by a visualization of a determined volume. In other words, the volume of at least the sections of the limb is determined from the 3D representation and the volume is output together with a fluorescence image and the visualization of the 3D representation. A visualization of the determined volume can be a graphic illustration, for example color scale illustration. However, a graphic illustration should also include the representation of the determined value as a numerical value.

The method of diagnosing lymphedema can further include the performance of the diagnosis on more than one limb. For example, the method can be executed on a left and a right limb of a patient. A differential diagnosis is thereby possible.

Furthermore, the method can be enhanced in that the fluorescent agent is administered to an arm or leg of a patient by injecting the fluorescent agent in tissue between phalanges of the foot or hand of the patient.

According to another example, there is a method of long-term therapy of lymphedema, comprising the steps of:
performing a diagnosis relative to lymphedema according to one or more of the above mentioned examples of this method,
   - performing a therapy on the patient, the therapy being adjusted to the diagnostic result relative to lymphedema,
repeating the steps of diagnosing lymphedema and performing a therapy on the patient, wherein in each iteration, the therapy is adjusted to the diagnosis of lymphedema, in particular to the severity or level of lymphedema.

The method of long-term therapy is particularly useful because the diagnosis of lymphedema provides, in contrast to traditional methods, objective results with respect to the severity or level of the disease. The success of a long-term therapy can therefore be analyzed from an objective point of view. Hence, the diagnosis and the therapy can be enhanced.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic illustration of an image capturing and processing device,
- Fig. 2: a schematic illustration of an image capturing device and a processing unit of the image capturing and processing device,
- Fig. 3: a simplified illustration of the operation of the image capturing device comprising the fluorescence imaging unit and the topology capturing unit,
- Fig. 4: a simplified illustration of an overlay image comprising a visualization of the 3D representation and the fluorescence image,
- Fig. 5a): an example of a visible light image,
- Fig. 5b): the corresponding fluorescence image,
- Fig. 6: a large overlay image, which is in part generated from the exemplary visible light in fluorescence images shown in Figs. 5a) and 5b),
- Fig. 7: a schematic illustration showing an internal prism assembly of the image capturing device,
- Fig. 8: a flow-chart of a stitching algorithm,
- Fig. 9: a schematic illustration showing another internal prism assembly of the image capturing device.

In the drawings, the same or similar types of elements or respectively corresponding parts are provided with the same reference numbers in order to prevent the item from needing to be reintroduced.

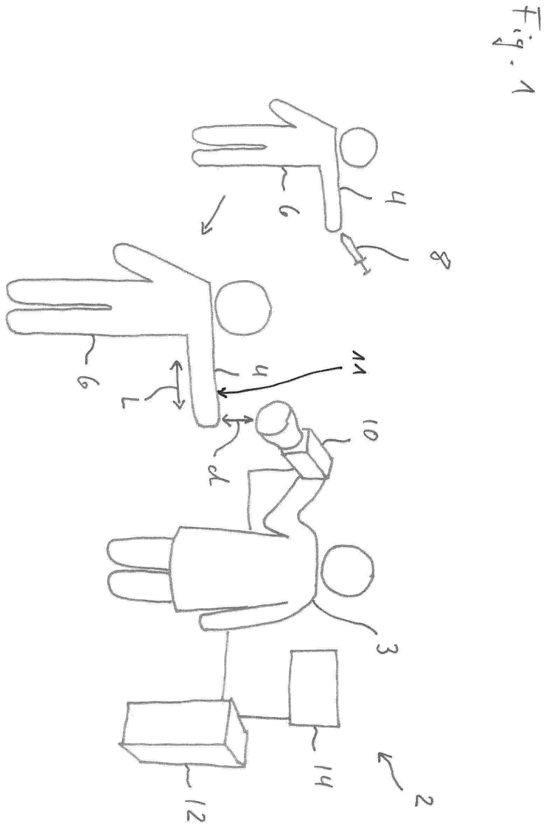

Fig. 1 illustrates an image capturing and processing device 2, which is configured to measure a fluorescence signal in a tissue of a limb 4 of a patient 6. By way of an example only, the limb 4 of the patient 6 is the arm. The measurement of the fluorescence signal can also be performed on other limbs 4 of the patient 6, for example at the legs.

Before the measurement initially starts, a fluorescent agent 8 is administered, i.e. injected, in the tissue of the patient's limb 4. The method for measuring a fluorescence signal in the tissue of the limb 4, which will also be explained when making reference to the figures illustrating the image capturing and processing device 2, excludes the step of administering the fluorescent agent 8.

The fluorescent agent 8 is for example ICG or methylene blue. ICG (Indocyanine Green) is a green colored medical dye that is used for over 40 years. ICG emits fluorescent light when exited with near infrared light having a wavelength between 600 nm and 800 nm. The emitted fluorescence light is between 750 nm and 950 nm. It is also possible that the fluorescent agent 8 comprises two different medical dyes. For example, the fluorescent agent 8 can be a mixture of methylene blue and ICG.

Subsequent to the administration of the fluorescent agent 8, as it is indicated by an arrow in Fig. 1, the patient's limb 4 is inspected using an image capturing device 10, which forms part of the image capturing and processing device 2.

The image capturing device 10 is configured to image a surface 11 of the limb 4 and to detect the fluorescence signal, which results from illumination of the fluorescent agent 8 with excitation light. For emission of light having a suitable excitation wavelength, the image capturing device 10 comprises an illumination unit 16 (not shown in Fig. 1).

The captured images are communicated to a processing device 12, which also forms part of the image capturing and processing device 2. The results of the analysis are output, for example displayed on a display 14 of the processing device 12. The image capturing device 10 can be handled by a physician 3.

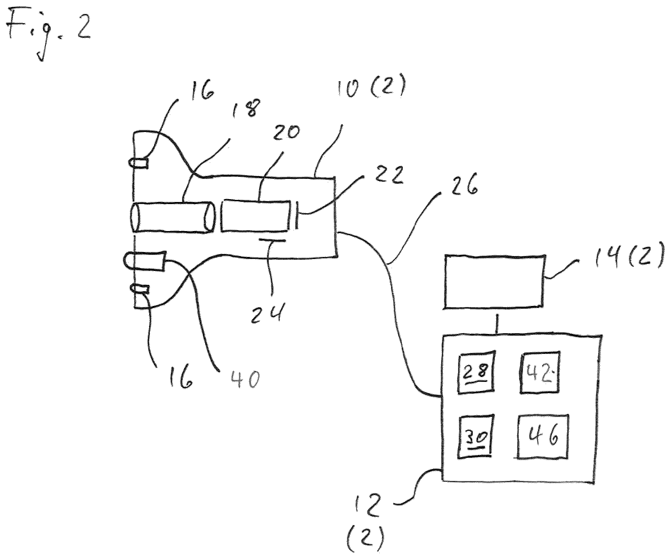

Fig. 2 is a schematic illustration showing the image capturing device 10 and the processing unit 12 of the image capturing and processing device 2 in more detail. The image capturing device 10 comprises an illumination unit 16 which is configured to illuminate the tissue with excitation light having a wavelength suitable to generate fluorescent light by exciting emission of the fluorescent agent 8. For example, a plurality of LEDs is provided in the illumination unit 16.

Furthermore, the image capturing device 10 comprises a topology capturing unit 40. The topology capturing unit 48 can be a line scanner or a LIDAR scanner, a point cloud generator or any other suitable device that can generate a 3D data set from an object.

The image capturing device 10 further comprises an objective lens 18 through which visible light and a fluorescence light are captured. Light is guided through the objective lens 18 to a prism assembly 20. The prism assembly 20 is configured to separate fluorescent light, which is in particular in a wavelength range between 750 nm and 950 nm, from visible light that results in the visible light image. The fluorescent light is directed on a fluorescence imaging unit 22, which is for example a CCD or CMOS sensor plus additional wavelength filters and electronics, if necessary. The fluorescence imaging unit 22 is configured to capture a fluorescence image by spatially resolved measurement of the emitted light, i.e. the excited emission of the fluorescent agent 8, so as to provide the fluorescence image. Furthermore, there is a visible light imaging unit 24, which can be another CCD or CMOS sensor plus an additional different wavelength filter and electronics, if necessary. The prism assembly 20 is configured to direct visible light on the visible light imaging unit 24 so as to allow the unit to capture the visible light image of a section of a surface 11 of the patient's limb 4. Similarly, the prism assembly 20 is configured to direct fluorescent light on the fluorescence imaging unit 22. The prism assembly 20, the fluorescence imaging unit 22 and the visible light imaging unit 24 will be explained in detail further below.

The visible light imaging unit 24 is an optional unit. In other words, the image capturing device 10 can be configured in that it only comprises the fluorescence imaging unit 22 and the topology capturing unit 40. Without prejudice and only for the sake of simplification of the explanations, reference will be made to an image capturing device 10 that comprises both units, namely the fluorescence imaging unit 22 and the visible light imaging unit 24.

The image capturing device 10 can also be 3D camera, which is suitable to capture a pair of stereoscopic images from which a 3D image including depth information can be calculated. In this case, the topology capturing unit 40 can be replaced by a processing unit that is capable of calculating data on the topology of the surface 11 of the patient's limb 4 from the 3D image data. In this case, no separate sensor for acquisition of data on the topology of the surface 11 of the limb 4 is required.

The topology capturing unit 40 is configured to capture data on the topology of the surface 11 of at least a section of the limb 4 of the patient 6. Furthermore, the topology capturing unit 40 is configured to determine the 3D representation of at least the section of the limb 4 from the captured data.

The image data and the data on the 3D representation is communicated from the image capturing device 10 to the processing device 12 via a suitable data link 26, which can be a wireless datalink or a wired data link, for example a data cable.

The processing device 12 comprises an output unit 42, which is configured to output the fluorescence image and a visualization of the 3D representation. The visualization of the 3D representation can be calculated in the processing device 12, for example by the output unit 42. The processing device 12 can also comprise a further unit, which is not separately shown, and which can be considered a part of the topology capturing unit 40. This unit is configured to calculate a visualization of the 3D representation from the data of the 3D representation that is captured for example of a scanning device of the topology capturing unit 40. The visualization of the 3D representation can be for example a point cloud, in a grid network structure, a 3D model rendering or any other suitable 3D illustration.

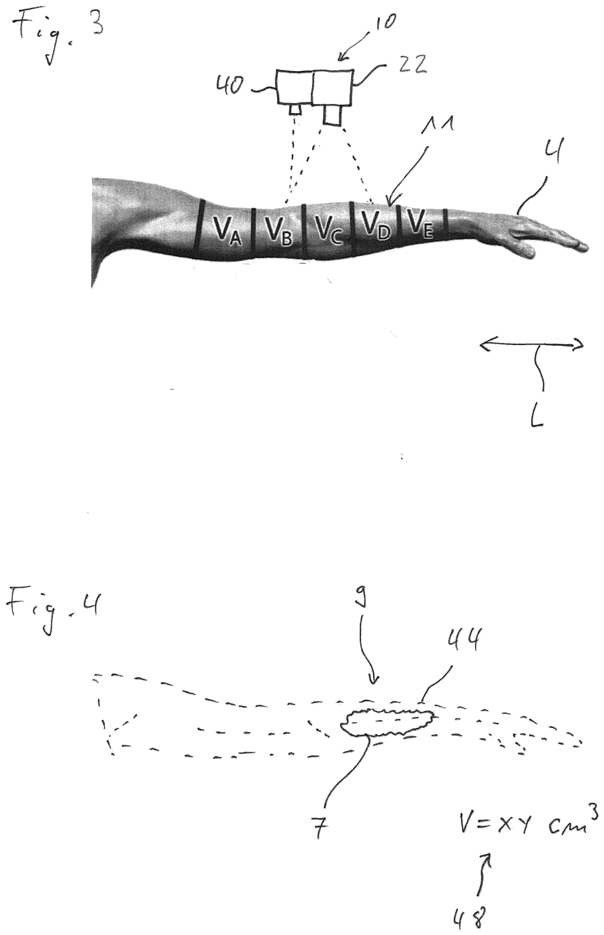

Fig. 3 illustrates a situation, in which the image capturing device 10 including the fluorescence imaging unit 22 and the topology capturing unit 14 captures a fluorescence image and data on the topology of the surface 11 of the limb 4. This is performed by moving the image capturing device 10 along the longitudinal direction L of the limb 4. The scanned data is processed by the output unit 42 and a visualization 44 of the 3D representation of the limb 4 is generated.

Fig. 4 illustrates such a visualization 44. By way of an example only, the visualization 44 is the point cloud. This visualization 44 of the limb 4 can be displayed on the display 14 of the processing device 12 (see Fig. 2).

Fig. 3 also illustrates the measurement of the topology of the surface 11 of the limb 4 in contrast it to the prior art. The traditional approach is to determine the volume of limb 4 of a patient by the so-called tape method. Tapes are placed for example around the arm of a patient in regular intervals. In Fig. 3, the tapes are illustrated as black lines. The circumference of the arm at the position of the tape is determined and the total volume is approximated by cylinders of frustums having a circumference that equals the measured circumferences at the respective positions of the tapes. This traditional method, however, is a very rough approximation. Furthermore, it suffers from inconsistencies and measurement errors. It is difficult to compare the values of different measurements, in particular if the measurements are performed at different points in time or by different persons.

Referring back to Fig. 4, it can be seen that from the 3D representation, which means the 3D data characterizing the topology of the surface 11 of the limb 4 of the patient 6, a volume V of the limb 4 can be calculated. For calculation of the volume V of the limb 4, the processing device 12 includes a volume determination unit 46. The volume determination unit 46 is configured to determine the volume V of at least a section of the limb 4 from the 3D representation. The volume V, which is has been calculated, can be displayed together with the visualization 44 of the 3D representation. For example, the volume V can be displayed using simple 8 digits, this is illustrated in Fig. 4, in which the visualization 48 of the determined volume is: "V=xy cm3".

Furthermore, the processing device 12 comprises a superimposing unit 30, which is configured to superimpose the fluorescence image and the visualization 44 of the 3D representation of the at least a section of the limb 4 so as to provide an overlay image 9. The output unit 42 is accordingly configured to output the overlay image 9.

This is illustrated in Fig. 4 by showing a cloudlike structure illustrating the fluorescence image 7. In this part of the limb 4, there is a high concentration of fluorescent agent, which is a strong indication of insufficient lymph transport. This information is merged together with the information on the volume V and the visualization 44 of the 3D representation of the patient's limb 4. This information all together forms the overlay image 9. It gives the user a very good database for a subsequent diagnosis of for example lymphedema.

Furthermore, the capturing of the fluorescence image 7 and the capturing of the data on the topology of the surface 11 of the section of the limb 4 and the subsequent determination of the 3D representation of the section of the limb 4 from the captured data can be performed for at least a first measurement series and for a measurement series. The different measurement a series can be performed on different limbs 4 or at different points in time. For example, the first measurement series can be performed on the left arm of the patient 6 and the second measurement series can be performed on the right arm of the patient 6. It is also possible that the first measurement series is performed during a first examination appointment and the second measurement series is performed during the second examination appointment, which is for example later in time (several weeks of month later). The output unit 42 is configured to output the fluorescence images and the visualizations of the 3D representations of the first and second series. The output unit 42 is further configured to generate and output a difference image, which is calculated from the captured data of the first and second series. In other words, the fluorescence images and the visualizations 44 of the 3D representations of the first and second series are output in that the differences between the first and second series are highlighted in the difference image. By outputting this information in the difference image, differences between the data of the first series and the second series can be easily visualized. For example a difference in Volume V between a left arm and a right arm of the patient 6. Typically, only one arm is affected by lymphedema. Hence, the amount or level of lymphedema can be easily visualized by this comparison. By comparing the results from the first examination appointment with the measurement of the second examination appointment the progress of the disease can be visualized or an effect of the therapeutic treatment can be visualized. In both cases, the user of the system is provided with a very good databases for further diagnosis and therapy.

Furthermore, the image capturing device 10 can be configured in that the fluorescence imaging unit 22 and the visible light imaging unit 24 are operated to simultaneously capture the visible light image and the fluorescence image. In particular, the image capturing device 10 does not perform time switching between the signal of the fluorescence image and the signal of the visible light image. In other words, the sensors of the fluorescence imaging unit 22 and the visible light imaging unit 24 are exclusively used for capturing images in the respective wavelength range, which means that the sensors of the imaging units 22, 24 are used for either capturing a fluorescence image in the **IR** spectrum or for capturing a visible light image in the visible spectrum. The sensors 22, 24 are not used for capturing images in both wavelength ranges. This results in significant advantages. For example, the sensors can be exactly positioned in focus, which is not possible when an image sensor is used for both purposes, i.e. to capture visible light and infrared light, because the focus point for these different wavelengths typically differ in position. Furthermore, the sensor parameters can be adjusted individually, for example with respect to a required exposure time or sensor gain. Individual settings are advantageous because **IR** signals are typically lower than visible light signals.

The fluorescence imaging unit 22 and the visible light imaging unit 24 have a fixed spatial relationship to each other. This is because the units are arranged in one single mounting structure or frame of the image capturing device 10. Furthermore, the fluorescence imaging unit 22 and the visible light imaging unit 24 use the same objective lens 18 and prism assembly 20 for imaging of the fluorescence image and the visible light image, respectively. Due to these measures, the fluorescence imaging unit 22 and the visible light imaging unit 24 are configured in that a viewing direction and a perspective of the fluorescence image and the visible light image are linked via a known and constant relationship. In the given embodiment, the viewing direction of the two images are identical because both units 22, 24 image via the same objective lens 18.

The image capturing device 10 can be further configured to operate the fluorescence imaging unit 22 and the visible light imaging unit 24 to repeat the capturing of the fluorescence image and the visible light image so as to provide a series of fluorescence images and a series of visible light images. At the same time, the topology capturing unit 40 captures data on the topology of the surface 11 of the limb 4. This operation can be performed by the processing device 12 operating the image sensor of the fluorescence imaging unit 22, the image sensor of visible light imaging unit 24 and the topology capturing unit 40. The series of images is typically captured while an operator or physician 3 (see Figs. 1 and 3) moves the image capturing device 10 along a longitudinal direction L of the limb 4 of the patient 6. This movement can be performed in that subsequent images of the series of images comprise overlapping parts. In other words, details which are shown in a first image of the series of images are also shown in a subsequent second image of the series. This is important for the subsequent stitching process. To safeguard that corresponding features can be found in subsequent images, the frequency of image acquisition can be set to a sufficiently high value. The capturing of the images can be manually initiated by for example the physician 3 or the capturing of images can be controlled by the image capturing device 10 in that the described prerequisite is fulfilled. This in particular pertains to the visible light images.

Once the two series of images (i.e. a first series of visible light images and a second series of fluorescence images) or the series of image pairs (each image pair comprising a fluorescence image and a visible light image) are captured by the capturing device 10 and received in the processing device 12, the series of visible light images can be processed by a stitching unit 28 (see Fig. 2). The stitching unit 28 is configured to apply a stitching algorithm on the series of visible light images to generate a large visible light image of the limb 4. The large image is "larger" in that it shows a greater section of the limb 4 of the patient 6, which is analyzed with the image capturing device 10, then a single image.

The stitching algorithm starts with stitching of the visible light images. The stitching algorithm generates and applies a set of stitching parameters when preforming the stitching operation. The detailed operation of the stitching unit 28 will be described further below. The stitching unit 28 is configured to apply the stitching algorithm not only on the series of visible light images but also on the series of fluorescence images so as to generate a large fluorescence image. The stitching algorithm, which is applied for stitching of the fluorescence images is the same algorithm which is used for stitching of the visible light images. Furthermore, the stitching of the fluorescence images is performed using the same set of stitching parameters which was determined when performing the stitching of the visible light images. This is possible, because there is a fixed relationship between the viewing direction and perspective of the visible light images and the fluorescence images. Naturally, if the viewing direction and perspective of the visible light images and the fluorescence images are not identical, a fixed offset or a shift in the stitching parameters has to be applied. This takes into account the known and fixed spatial relationship between the **IR** and Vis image sensors and the corresponding optics.

Subsequent to the stitching, the large visible light image and the large fluorescence image are output together with the visualization of the 3D representation 44. For example, the images and the visualization of the 3D representation 44 are displayed side-by-side on the display 14. Unlike traditional inspection systems, the display 14 shows a visible light image and a fluorescence image that correspond to each other. In other words, details that can be seen on the fluorescence image, for example a high fluorescence intensity that indicates an accumulation of lymphatic fluid, can be found in the patient's limb 4 exactly on the corresponding position, which is shown in the visible light image. This enables the physician 3 to exactly spot areas in which an accumulation of lymphatic fluid is present. This is very valuable information for example for a tailored and specific therapy of the patient 6.

It is also possible that the visible light image, the fluorescence image and the visualization of the 3D representation 44, in particular the large visible light image and the large fluorescence image together with the 3D representation 44 are superimposed so as to provide an overlay image, in particular in a large overlay image, of the limb 4. This can be performed by a superimposing unit 30 of the processing device 12. The overlay image can also be output via the display 14.

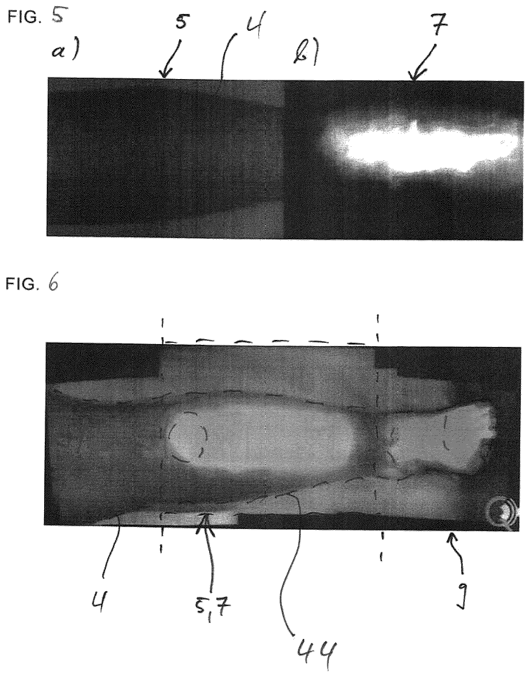

Fig. 5a) shows an example of a visible light image 5, in which a section of a surface 11 of the limb 4 of the patient 6 is visible. By way of an example only, a section of the patient's leg is depicted. Fig. 5b) shows the corresponding fluorescence image 7 determined by measuring the fluorescence signal of the fluorescence agent 8, which has been applied to the patient's tissue in the leg. A high-intensity spot or area of the fluorescence signal is visible. This strongly indicates an accumulation of lymph, which is due to a slow lymphatic transport and a possible lymphedema in the patient's leg. Advantageously, the physician 3 can locate the area, in which the slow lymphatic transport takes place by comparing the fluorescence image 7 with the visible light image 5.

In Fig. 6, there is the overlay image 9, wherein in addition to the images shown in Figs. 5a) and 5b), stitching of the visible light images 5 and fluorescence images 7 has been performed. Furthermore, the overlay image 9 includes the visualization of the 3D representation 44. This visualization is similar to the visualization 44 that has been explained with reference to Fig. 4. By way of an example only, the overlay image 9, which is shown in Fig. 6 dispenses with the visualization of the volume 48.

An exemplary single visible light image 5 and fluorescence image 7 can also be seen in Fig. 6, it respectively projects between the straight dashed lines shown in the large overlay image 9. By stitching together the visible light images 5 and the fluorescence images 7, the large overlay image 9 showing almost the entire limb 4 of the patient 6 can be provided. The fluorescence signal can be shown in false color so as to clearly distinguish from features of the visible light image 5.

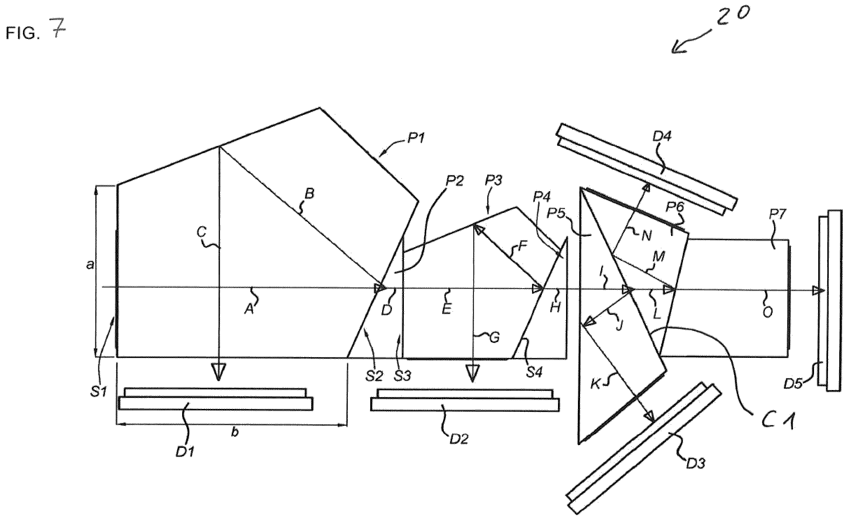

In Fig. 7, there is an embodiment of the prism assembly 20 of the image capturing device 10. This pertains to an embodiment in which the image capturing device 10 is configured to simultaneously acquire an visible light image 5 and fluorescence image 7. A first prism P1 is a pentagonal prism. The incoming light beam A, which is visible light and fluorescence light, enters the first prism P1 via the entrance face S1 and is partially reflected on face S2, being one of the two faces not adjoining the entrance face S1. The reflected beam B is then reflected against a first one of the faces adjoining the entrance face S1. The angle of reflection can be below the critical angle, so that the reflection is not internal (the adjoining face can be coated to avoid leaking of light and reflect the required wavelength of interest). The reflected beam C then crosses the incoming light beam A and exits the first prism P1 through the second one of the faces adjoining the entrance face S1, towards sensor D1. A part of the beam A goes through face S2 and enters compensating prism P2. Two non-internal reflections can be used to direct the incoming beam A via beams B and C towards the sensor D1. Furthermore, there can be no air gaps between prisms P1 and P2 and no air gaps between prisms P3 and P4 and no air gaps between prisms P2 and P3. Prism P2 is a compensator prism which is for adjusting the individual length of the light paths from the entrance face S1 to the sensors D1..D5.

From P2, the beam D enters a second pentagonal prism P3. As in prism P1, inward reflection is used to make the beam cross itself. For brevity, the description of the beam will not be repeated, except to state that in prism P3, the beam parts E, F and G correspond to beam parts A, B and C in prism P1, respectively. Prism P3 can also not use internal reflection to reflect the incoming beam towards sensor D2. Two non-internal reflections can be used to direct the incoming beam E via beams F and G towards sensor D2.

After prism P3, there is another compensating prism P4. Finally, beam H enters the dichroic prism assembly comprising prisms P5, P6, and P7, with sensors D3, D4 and D5 respectively. The dichroic prism assembly is for splitting visible light in red, green and blue components towards respective sensors D3, D4 and D5. The light enters the prism assembly through beam I. Between P5 and P6, an optical coating C1 is placed and between prisms P6 and P7 another optical coating C2 is placed. Each optical coating C1 and C2 has a different reflectance and wavelength sensitivity. At C1, the incoming beam I is partially reflected back to the same face of the prism as through which the light entered (beam J). At that same face, the beam, now labelled K, is once again reflected towards sensor D3. The reflection from J to K is an internal reflection. Thus, sensor D3 receives light reflected by coating C1, and in analogue fashion sensor D4 receives light from beam L reflected by coating S2 (beams M and N), and sensor D5 receives light from beam O that has traversed the prism unhindered.

Between prism P4 and prism P5 there is an air gap. In the prism assembly 20, the following total path lengths can be defined for each endpoint channel (defined in terms of the sensor at the end of the channel):
Sensor D1 (e.g. first near infrared) path: A + B + C
Sensor D2 (e.g. second near infrared) path: A + D + E + F + G
Sensor D3 (e.g. red) path: A + D + E + H + I + J + K
Sensor D4 (e.g. blue) path: A + D + E + H + I + 0
Sensor D5 (e.g. green) path: A + D + E + H + I + M+ N

The path lengths are matched, so that A + B + C = A + D + E + F + G = A + D + E + H + I + J + K = A + D + E + H + I + O = A + D + E + H + I + M + N.

The matching of path lengths can comprise an adjustment for focal plane focus position differences in wavelengths to be detected at the sensors D1 - D5. That is, for example the path length towards the sensor for blue (B) light may not be exactly the same as the path length towards the sensor for red (R) light, since the ideal distances for creating a sharp, focused image are somewhat dependent on the wavelength of the light. The prisms can be configured to allow for these dependencies. D + H lengths can be adjusted and act as focus compensators due to wavelength shifts, by lateral displacement of the compensator prisms P2, P4.

A larger air gap in path I can be used for additional filters or filled with a glass compensator for focus shifts and compensation. An air gap needs to exist in that particular bottom surface of red prism because of the internal reflection in the path from beam J to beam K. A space can be reserved between the prism output faces and each of the sensors D1 - D5 to provide an additional filter, or should be filled up with glass compensators accordingly.

The sensors D1 and D2 are IR sensors, configured for capturing the fluorescence image 7. By way of an example, the sensors D1 and D2 plus suitable electronics are a part of the fluorescence imaging unit 22. The sensors D3, D4 and D5 are for capturing the three components of the visible light image 5. By way of an example, the sensors D3, D4 and D5 plus suitable electronics are a part of the visible light imaging unit 24. It is also possible to consider the corresponding prisms that direct the light beams on the sensors a part of the respective unit, i.e. the fluorescence imaging unit 22 and the visible light imaging unit 24, respectively.

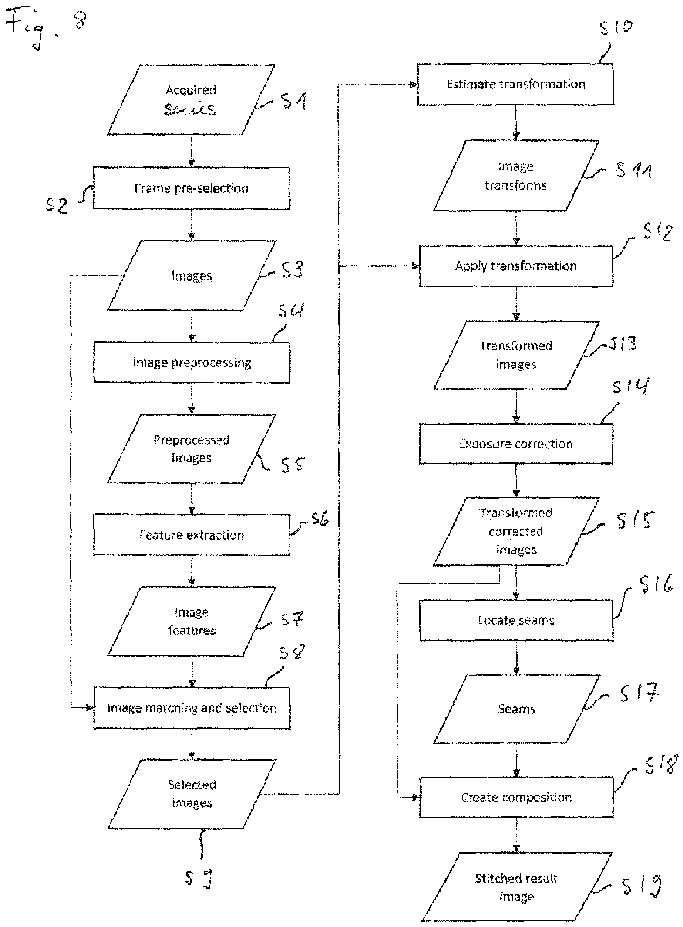

Fig. 8 shows a flowchart of the stitching algorithm, which can be used for stitching of the visible light images and the fluorescence images. The flow chart is more or less self-explanatory and will be very briefly described. Firstly, the acquired series of images (S1) is forwarded to the stitching unit 24 of the processing device 12. The algorithm then performs a frame preselection (step S2). In this preselection step, frames suitable for stitching are selected. S3 represents the selected images to be stitched, they then undergo preprocessing (step S4). In the preprocessed images (S5) a feature extraction is performed (step S6). When the image features have been extracted (S7), image matching (step S8) is performed using the images known from S3 and the extracted features from step S7. Based on the selected images (S9) a transformation of the images is estimated (step S10). This estimation of image transformation (S11), also referred to as stitching parameters, is applied (step S12). The application of the transformation results in transformed images (S13). A further image correction can be performed, for example an exposure correction (step S14). The transformed and corrected images (S15) are stitched together by locating seams (step S16), i.e. lines along which the images are joined together. The data indicating the location of the seams (S17) is used together with the transformed and corrected images (S12) to create a composition of images (step S18). In the given embodiment, this results in the large visible light image or the large fluorescence image, these are the stitching results (S19).

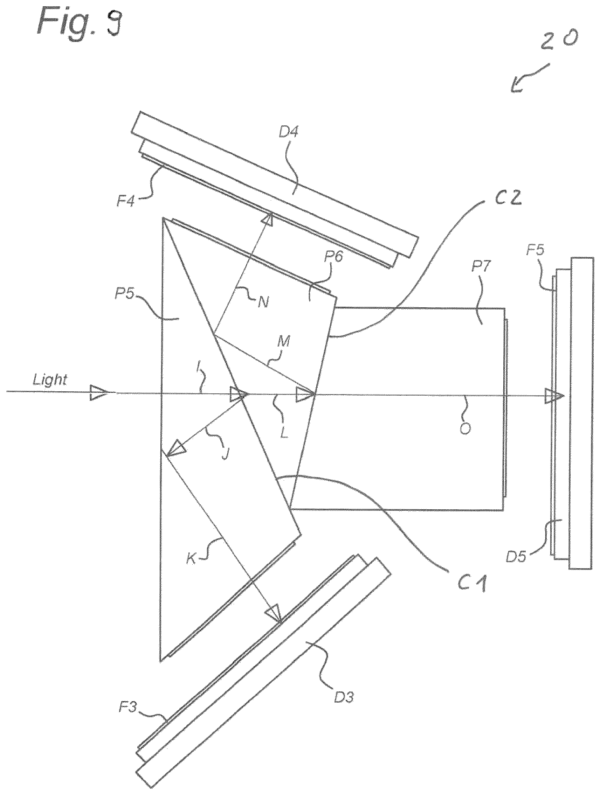

In Fig. 9, there is an embodiment of another prism assembly 20 of the image capturing device 10. The prism assembly 20 comprising prisms P5, P6, and P7, which, for example, are configured for splitting light in red, green and blue components towards respective sensors D3, D4, and D5. According to a further embodiment, the prism assembly 20 is configured to split incoming light in a green component, a red/blue component and an infrared component and to direct these towards the respective sensors D3, D4, and D5. According to still another embodiment, the prism assembly 20 is configured to split incoming light in a visible light component, which is directed to a red/green/blue sensor (RGB sensor), a first infrared component of a first wavelength or wavelength interval and a second infrared component of a second wavelength or wavelength interval, and to direct these towards the respective sensors D3, D4, and D5.

The light enters the prism assembly 20 through the arrow indicated. Between P5 and P6, an optical coating C1 is placed and between prisms P6 and P7 an optical coating C2 is placed, each optical coating C1 and C2 having a different reflectance and wavelength sensitivity. At C1, the incoming beam I is partially reflected back to the same face of the prism P5 as through which the light entered (beam J). At that same face, the beam, now labelled K, is once again reflected towards filter F3 and sensor D3. The reflection from J to K is an internal reflection. Thus, filter F3 and sensor D3 receive light reflected by coating C1, and in analogue fashion filter F4 and sensor D4 receive light from beam L reflected by coating S2 (beams M and N). Filter F5 and sensor D5 receives light from beam O that has traversed the prisms unhindered.

When making reference to the embodiment in which the incoming light is split up in a red, green and blue component, the coatings and filters are selected accordingly.

In the embodiment, in which the incoming light is separated in a green component, a red/blue component and an infrared component, the filter F3 can be a patterned filter (red/blue). In particular, there is an array of red and blue filters in an alternating pattern. The pattern can consist of groups of 2x2 pixels, which are filtered for one particular color. Filter F4 can be a green filter, which means the filter comprises only green filters. There is a single pixel grid with the light received at each pixel being filtered with a green filter. Filter F5 can be an IR filter. Each pixel is filtered with an IR filter.

In general, the coatings C1, C2 should match the filters F3, F4, F5. For example, the first coating C1 may transmit visible light while reflecting IR light, so that IR light is guided towards IR filter F3. The second coating C2 may be transparent for green light while reflecting red and blue light, so that filter F4 should be the red/blue patterned filter and F5 should be the green filter 23.

According to the further embodiment, in which incoming light is split up in the visible light component (RGB), the first infrared component and the second infrared component, the coatings C1, C2 and the filters F3, F4, F5 are configured in that for example the sensor D4 is a color sensor (RGB sensor) for detecting the visible light image in all three colors. Furthermore, the sensor D3 can be configured for detecting fluorescence light of the first wavelength and the sensor D5 is configured for detecting fluorescence light of the second wavelength.

Similarly, when making reference to the prism assembly 20 in Fig. 7, the coatings S1, S2, S3, S4, C1 and C2 as well as the filters F1, F2, F3, F4 and F5, which are arranged in front of a respective one of the sensors D1, D2, D3, D4 and D5, can be configured in that up to four fluorescence light wavelengths can be detected. For example, the sensor D4 is a color sensor for detecting the visible light image in all three colors. The sensor D3 is for detecting fluorescence light of a first wavelength or wavelength interval, the sensor D5 is for detecting fluorescence light of a second wavelength or wavelength interval, the sensor D1 is for detecting fluorescence light of a third wavelength or wavelength interval, and the sensor D2 is for detecting fluorescence light of a fourth wavelength or wavelength interval.

Further embodiments:
Embodiment 1: A method of measuring a fluorescence signal in a tissue of a limb (4), to which a fluorescent agent (8) has been added, and of determining a 3D representation of at least a section of the limb (4), wherein the tissue to which the fluorescent agent (8) has been added forms part of the limb (4), the method comprising the steps of:
   - capturing a fluorescence image (7) by illuminating the tissue with excitation light having a wavelength suitable to generate emitted light by excited emission of the fluorescent agent (8), and by spatially resolved measurement of the emitted light so as to provide the fluorescence image (7),
   - capturing data on a topology of the surface (11) of at least the section of the limb (4) and determining a 3D representation of at least the section of the limb (4) from the captured data,
   - outputting the fluorescence image (7) and a visualization (44) of the 3D representation.
Embodiment 2: The method of embodiment 1, further comprising the steps of:
   - determining a volume of at least the section of the limb (4) from the 3D representation and
   - outputting the fluorescence image (7) and the visualization (44) of the 3D representation together with a visualization (48) of the determined volume (V).
Embodiment 3: The method of any of embodiments 1 or 2, further comprising the steps of:
   - superimposing the fluorescence image (7) and the visualization (44) of the 3D representation of at least the section of the limb (4) so as to provide an overlay image (9) and
   - outputting the overlay image (9) as the output of the fluorescence image (7) and the visualization (44) of the 3D representation.
Embodiment 4: The method of any of embodiments 1 to 3, wherein the steps of capturing the fluorescence image (7) and of capturing data on the topology of the surface (11) of at least the section of the limb (4) are performed simultaneously.
Embodiment 5: The method of any of embodiments 1 to 4, wherein the steps of capturing the fluorescence image (7) and data on the topology of the surface (11) of at least the section of the limb (4) and the determination of the 3D representation of at least the section of the limb (4) from the captured data, are performed in at least a first measurement series and a second measurement series, wherein the different measurement series are performed on different limbs (4) or at different points in time, and wherein the step of outputting includes outputting the fluorescence images (7) and the visualizations (4) of the 3D representations of the first and second series, wherein in particular the fluorescence images (7) and the visualizations (4) of the 3D representation of the first and second series are output as at least one difference image.
Embodiment 6: The method of any of embodiments 1 to 5, further comprising the steps of:
   - capturing a visible light image (5) of at least the section of the surface of the limb (4), wherein a viewing direction and/or a perspective of the fluorescence image (7) and the visible light (5) image are linked via a known relationship and
   the step of outputting includes:
   - outputting the visible light image (5) together with the fluorescence image (7) and the visualization (44) of the 3D representation,
   wherein in particular, the method further comprising:
   - repeating the steps of capturing of the fluorescence image (7) and capturing of the visible light image (5) to provide a series of fluorescence images (7) and a series of visible light images (5), wherein the capturing of data on the topology of the surface of the limb (4) is performed in at least the section of the limb (4) that is imaged when capturing the series of fluorescence images (7) and the visible light images (5),
   - applying a stitching algorithm on the series of visible light images (7) to generate a large visible light image, wherein the stitching algorithm determines and applies a set of stitching parameters,
   - applying the stitching algorithm on the series of fluorescence images (7) to generate a large fluorescence image, wherein the stitching algorithm applies the set of stitching parameters determined when performing the stitching of the visible light images (5), wherein
   the step of outputting includes
   - outputting the large visible light image (5) together with the large fluorescence image (7) and the visualization (44) of the 3D representation.
Embodiment 7: The method according to any of embodiment 1 to 6, wherein the measurement of the fluorescence signal is performed on a tissue, to which at least a first and a second fluorescent agent (8) has been added, wherein the step of capturing the fluorescence image comprises:
   - capturing a first fluorescence image in a first wavelength range, which is generated by illuminating the tissue with first excitation light having a first wavelength suitable to generate emitted light by a first excited emission of the first fluorescent agent (8), and
   - capturing a second fluorescence image in a second wavelength range, which is generated by illuminating the tissue with second excitation light having a second wavelength suitable to generate emitted light by a second excited emission of the second fluorescent agent (8), and the step of outputting comprises outputting the first and the second fluorescence image and the visualization (44) of the 3D representation.
Embodiment 18: A method of diagnosing lymphedema, comprising the steps of:
   - administering a fluorescent agent (8) to a limb (4),
   - measuring a fluorescence signal in a tissue of the limb (4), to which the fluorescent agent (8) has been administered, and determining a 3D representation of at least a section of the limb (4), wherein the tissue to which the fluorescent agent (8) has been added forms part of the limb (4),
   - capturing a fluorescence image (7) by illuminating the tissue with excitation light having a wavelength suitable to generate emitted light by excited emission of the fluorescent agent (8), and by spatially resolved measurement of the emitted light so as to provide the fluorescence image (7),
   - capturing data on a topology of the surface of at least the section of the limb (4) and determining a 3D representation of at least the section of the limb (4) from the captured data,
   - outputting the fluorescence image (7) and a visualization (44) of the 3D representation,
   - deriving a diagnostic result relative to lymphedema, in particular relative to a severity or level of lymphedema, by analyzing the fluorescence image (7) and the visualization (44) of the 3D representation.
Embodiment 19: The method according to embodiment 18, wherein the fluorescent agent (8) is administered to an arm or leg of a patient by injecting the fluorescent agent (8) in tissue between phalanges of the foot or hand of the patient (6).
Embodiment 20: A method of long-term therapy of lymphedema, comprising the steps of:
   performing a diagnosis relative to lymphedema by performing the steps of embodiment 18 or 19 on a patient (6),
   - performing a therapy on the patient (6), the therapy being adjusted to the diagnostic result relative to lymphedema, repeating the steps of diagnosing lymphedema and performing a therapy on the patient (6), wherein in each iteration, the therapy is adjusted to the diagnosis of lymphedema, in particular to the severity or level of lymphedema.

### List of References

- 2: image capturing and processing device
- 3: physician
- 4: limb
- 5: visible light image
- 6: patient
- 7: fluorescence image
- 8: fluorescent agent
- 9: overlay image
- 10: image capturing device
- 11: surface
- 12: processing device
- 14: display
- 16: illumination unit
- 18: objective lens
- 20: prism assembly
- 22: fluorescence imaging unit
- 24: visible light imaging unit
- 26: data link
- 28: stitching unit
- 30: superimposing unit
- 40: topology capturing unit
- 42: output unit
- 44: visualization of a 3D representation
- 46: volume determination unit
- 48: visualization of volume

- P1: first pentagonal prism
- P2, P4: compensating prism
- P3: second pentagonal prism
- P5, P6, P7: dichroic prism assembly
- A: incoming light beam
- B..O: light beams
- S1: entrance face
- D1..D5: sensors
- C1, C2: coating

- L: longitudinal direction
- V: volume

## Claims

1. An image capturing and processing device (2) configured to measure a fluorescence signal in a tissue of a limb (4), to which a fluorescent agent (8) has been added, and to determine a 3D representation of at least a section of the limb (4), wherein the tissue to which the fluorescent agent (8) has been added forms part of the limb (4), the device (2) comprising an image capturing device (10), which comprises:
- an illumination unit (16) configured to illuminate the tissue with excitation light having a wavelength suitable to generate emitted light by excited emission of the fluorescent agent (8),
- a fluorescence imaging unit (22) configured to capture a fluorescence image by spatially resolved measurement of the emitted light so as to provide the fluorescence image (7),
- a topology capturing unit (40) configured to capture data on a topology of the surface (11) of at least the section of the limb (4) and to determine a 3D representation of at least the section of the limb (4) from the captured data, **characterized by** a volume determination unit (46) configured to determine a volume (V) of at least the section of the limb (4) from the 3D representation,
the image capturing and processing device (2) further comprising a processing device (12), which comprises
- an output unit (42) configured to output the fluorescence image (7) and a visualization of the 3D representation together with a visualization (48) of the determined volume (V).

2. The device (2) of claim 1, wherein the processing device (12) further comprises:
- a superimposing unit (30) configured to superimpose the fluorescence image (7) and the visualization of the 3D representation of at least the section of the limb (4) so as to provide an overlay image (9) and
- the output unit (42) is configured to output the overlay image (9) as the output of the fluorescence image (7) and the visualization (44) of the 3D representation.

3. The device (2) of claim 1 or 2, wherein
the fluorescence imaging unit (22) and the topology capturing unit (40) are configured to simultaneously perform capturing of the fluorescence image (7) and capturing of data on the topology of the surface (11) of at least the section of the limb (4).

4. The device (2) of any of claims 1 to 3, wherein the image capturing device (10) further comprises:
- a visible light imaging unit (24) configured to capture a visible light image of at least the section of the surface of the limb (4), wherein the fluorescence imaging unit (22) and the visible light imaging unit (24) are configured in that a viewing direction and/or a perspective of the fluorescence image and the visible light image are linked via a known relationship, and
- the output unit (42) is configured to output the visible light image (5) together with the fluorescence image (7) and the visualization (44) of the 3D representation
wherein in particular
the fluorescence imaging unit (22) and the visible light imaging unit (24) are further configured to repeat capturing of the fluorescence image (7) and the visible light image (5) to provide a series of fluorescence images (7) and a series of visible light images (5), wherein the topology capturing unit is configured to capture data on the topology of the surface of the limb (4) in at least the section of the limb (4) that is imaged when capturing the series of fluorescence images (7) and the visible light images (5), the processing device (12) further comprising:
- a stitching unit (28) configured to apply a stitching algorithm on the series of visible light images (5) to generate a large visible light image of the limb (4), the stitching algorithm determining and applying a set of stitching parameters, wherein the stitching unit (28) is further configured to apply the stitching algorithm on the series of fluorescence images (7) to generate a large fluorescence image, wherein the stitching algorithm applies the set of stitching parameters determined when performing the stitching of the visible light images (5), and wherein
the output unit (42) is configured to output the large visible light image together with the large fluorescence image and the visualization (44) of the 3D representation.

5. The device (2) according to claim 4, wherein the fluorescence imaging unit (22) and the visible light imaging unit (24) are configured in that the viewing direction and the perspective of the fluorescence image (7) and the visible light image (5) are identical, wherein in particular the fluorescence imaging unit (22) and the visible light imaging unit (24) are configured in that the fluorescence image (7) and the visible light image (5) are captured through one and the same objective lens (18).

6. The device (2) according to claim 4 or 5, wherein the fluorescence imaging unit (22) and the visible light imaging unit (24) are configured to capture the fluorescence image (7) and the visible light image (5) simultaneously, in absence of time-switching between a signal of the fluorescence image (7) and a signal of the visible light image (5).

7. The device (2) according to any of claims 1 to 6, wherein the image capturing device (10) further comprises: a dichroic prism assembly (20) configured to receive fluorescent light forming the fluorescence image (7) and visible light forming the visible light image (5) through an entrance face (S1), comprising: a first prism (P1), a second prism (P3), a first compensator prism (P2) located between the first prism (P1) and the second prism (P3), a further dichroic prism assembly (P5, P6, P7) for splitting the visible light in three light components, and a second compensator prism (P4) located between the second prism (P3) and the further dichroic prism assembly (P5, P6, P7),
wherein the first prism (P1) and the second prism (P3) each have a cross section with at least five corners, each corner having an inside angle of at least 90 degrees, wherein the corners of the first prism (P1) and the second prism (P3) each have a respective entrance face (S1, S2) and a respective exit face, and are each designed so that an incoming beam which enters the entrance face (S1, S3) of the respective prism (P1, P3) in a direction parallel to a normal of said entrance face (S1, S3) is reflected twice inside the respective prism (P1, P3) and exits the respective prism (P1, P3) through its exit face parallel to a normal of said exit face,
wherein the normal of the entrance face (S1, S3) and the normal of the exit face of the respective prism (P1, P3) are perpendicular to each other; and
wherein, when light enters the first prism (P1) through the entrance face (S1), the light is partially reflected towards the exit face of the first prism (P1) thereby traveling a first path length from the entrance face (S1) of the first prism (P1) to the exit face of the first prism (P1 ), and the light partially enters the second prism (P3) via the first compensator prism (P2) and is partially reflected towards the exit face of the second prism (P3), thereby traveling a second path length from the entrance face of the first prism (P1) to the exit face of the second prism (P3),
wherein the first prism (P1) is larger than the second prism (P3) so that the first and the second path lengths are the same.
